(19) Europäisches Patentamt
European Patent Office
Office européen des brevets

(11) Veröffentlichungsnummer: **0 006 451**
**B1**

(12) # EUROPÄISCHE PATENTSCHRIFT

(45) Veröffentlichungstag der Patentschrift: **14.04.82**

(51) Int. Cl.³: **C 07 D 487/04,** A 61 K 31/415 // (C07D487/04, 235/00, 235/00)

(21) Anmeldenummer: **79101590.2**

(22) Anmeldetag: **23.05.79**

(54) **Neue Imidazo(1,2-a)imidazole, deren Säureadditionssalze, diese enthaltende Arzneimittel und Verfahren zu deren Herstellung.**

(30) Priorität: **23.06.78 DE 2827617**

(43) Veröffentlichungstag der Anmeldung: **09.01.80 Patentblatt 80/1**

(45) Bekanntmachung des Hinweises auf die Patenterteilung: **14.04.82 Patentblatt 82/15**

(84) Benannte Vertragsstaaten: **AT BE CH DE FR GB IT LU NL SE**

(56) Entgegenhaltungen:
DE-A-2109524
DE-A-2118261
DE-A-2361188

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim (DE)**
Erfinder: **Köppe, Herbert, Dr., Neuweg 72, D-6507 Ingelheim (DE)**
Erfinder: **Kummer, Werner, Dr., Georg-Scheuing-Strasse 15, D-6507 Ingelheim (DE)**
Erfinder: **Kobinger, Walter, Prof. Dr., Belghofergasse 27, A-1120 Wien (AT)**
Erfinder: **Lillie, Christian, Dr. Mag., Hansi-Niese-Weg 12, A-1130 Wien (AT)**
Erfinder: **Pichler, Ludwig, Dr., Gusshausstrasse 24, A-1040 Wien (AT)**

**EP 0 006 451 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Neue Imidazo-[1,2-a]-imidazole, deren Säureadditionssalze, diese enthaltende Arzneimittel und Verfahren zu deren Herstellung.

In der DE-A Nr. 2118261 sind substituierte Imidazo-[1,2-a]-imidazole, -pyrimidine und -diazepine offenbart, die hauptsächlich blutdrucksenkende Eigenschaften besitzen.

Die DE-A Nr. 2109524 beschreibt 2,3-Dihydrodioxoimidazo-[1,2-a]-imidazole und -pyrimidine, die auch blutdrucksenkende und daneben neuroleptische Wirkung entfalten.

Es wurde nunmehr überraschenderweise gefunden, dass bisher in der Literatur nicht vorbeschriebene, den Derivaten des Standes der Technik strukturähnliche Imidazo-[1,2-a]-imidazole eine bradycarde Wirkung haben.

Die Erfindung betrifft dementsprechend neue substituierte Imidazo-[1,2-a]-imidazolderivate der allgemeinen Formel I:

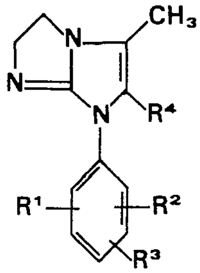

I

sowie deren physiologisch verträgliche Säureadditionssalze mit wertvollen therapeutischen Eigenschaften.

In der Formel I bedeuten $R^1$, $R^2$ und $R^3$, die gleich oder verschieden sein können, ein Wasserstoffatom oder ein Fluor-, Chlor- oder Bromatom oder eine Methyl- oder Trifluormethylgruppe, wobei mindestens einer der Reste $R^1$, $R^2$ oder $R^3$ nicht Wasserstoff sein darf. $R^4$ bedeutet ein Wasserstoffatom oder eine Methylgruppe.

Die Herstellung der Verbindungen der Formel I kann nach verschiedenen Verfahren erfolgen, wobei sich die folgenden besonders bewährt haben:

a) Umsetzung eines substituierten Phenyliminoimidazolidins der allgemeinen Formel II:

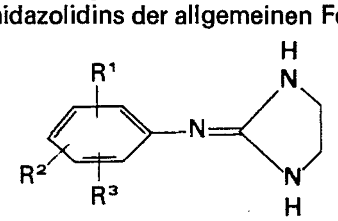

II

worin $R^1$ bis $R^3$ die oben genannten Bedeutungen besitzen, mit einer Oxoverbindung der allgemeinen Formel III:

$R^4 - CH - C - CH_3$
$\quad\;\; | \quad\;\; \|$
$\quad\;\; X \quad\; O$ III

worin $R^4$ wie oben angegeben definiert ist und X ein Chlor-, Brom- oder Jodatom bedeutet.

Die Umsetzung nach Verfahren a erfolgt zweckmässigerweise durch Erhitzen der Reaktionspartner — vorzugsweise in Gegenwart eines polaren oder unpolaren organischen Lösungsmittels — auf Temperaturen von 60 bis 180°C. Die speziellen Reaktionsbedingungen hängen im starken Masse von der Reaktivität der Umsetzungsteilnehmer ab. Die Umsetzung kann in Gegenwart eines säurebindenden Mittels wie z.B. Triäthylamin durchgeführt werden.

b) Cyclisierung eines 2-[N-Progargyl-N-phenylamino]-imidazolins-(2) der allgemeinen Formel IV:

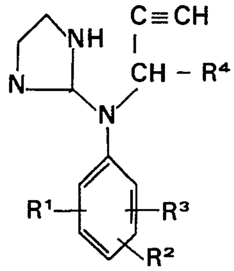

IV

worin $R^1$ bis $R^4$ die oben genannten Definitionen besitzen.

Bei dem Verfahren b wird ebenfalls bei erhöhter Temperatur — vorzugsweise zwischen 50 bis 150°C — gearbeitet. Als Lösungsmittel kommen sowohl polare als auch unpolare Solventien in Frage. Es empfiehlt sich, die Umsetzung in Gegenwart einer organischen Base wie z.B. Trimethylbenzylammoniumhydroxid durchzuführen.

c) Wasserabspaltung aus einem 7-Phenyl-5-hydroxy-5-methyl-2,3,5,6-tetrahydroimidazo-[1,2-a]-imidazol der allgemeinen Formel V:

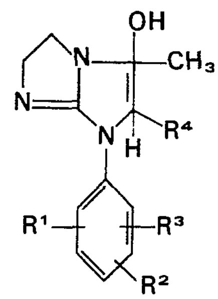

V

worin die Reste $R^1$ bis $R^4$ die oben angegebenen Bedeutungen haben, bei erhöhter Temperatur und/oder in Gegenwart wasserabspaltender Mittel.

Die Wasserabspaltung nach Verfahren c, welche bei Temperaturen zwischen 60 bis 180°C erfolgt, kann sowohl in Lösung als auch ohne Verwendung eines Lösungsmittels durchgeführt werden.

d) Umsetzung eines 1-Phenyl-2-imino-4-methylimidazolins-(4) der allgemeinen Formel VI:

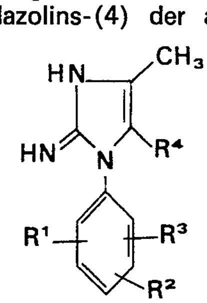

VI

worin $R^1$ bis $R^4$ wie oben angegeben definiert sind, mit 1,2-Dibromäthan.

Die Synthese nach Verfahren d wird ebenfalls bei erhöhter Temperatur — vorzugsweise bei 80 bis 180°C — durchgeführt. Lösungsmittel können angewendet werden, sind jedoch nicht erforderlich.

Ausgangsverbindungen der Formel II sind z.B. in den belgischen Patentschriften Nrn. 623305, 687657 und 705944 beschrieben. Ausgangsverbindungen der Formel III sind handelsübliche Verbindungen und aus der Literatur bekannt. Ausgangsverbindungen der Formel IV sind z.B. in der deutschen Offenlegungsschrift Nr. 2523103 beschrieben worden.

Substanzen der Formel V können durch Umsetzung von Phenyliminoimidazolidinen der Formel II mit Verbindungen der Formel III bei niederer Temperatur gewonnen werden. Ausgangsverbindungen der Formel VI erhält man bei der Umsetzung von Guanidinen der Formel VII:

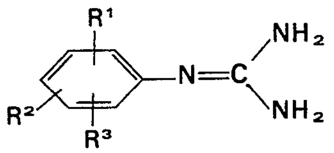

VII

mit Verbindungen der Formel III.

Die erfindungsgemässen Imidazo-[1,2-a]-imidazole der allgemeinen Formel I können auf übliche Weise in ihre physiologisch verträglichen Säureadditionssalze überführt werden. Zur Salzbildung geeignete Säuren sind beispielsweise Mineralsäuren, wie Salzsäure, Bromwasserstoffsäure, Jodwasserstoffsäure, Fluorwasserstoffsäure, Schwefelsäure, Phosphorsäure, Salpetersäure oder organische Säuren wie Essigsäure, Propionsäure, Buttersäure, Capronsäure, Caprinsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, Äthanphosphonsäure, 8-Chlortheophyllin.

Die neuen Verbindungen der Formel I sowie deren physiologisch verträgliche Säureadditionssalze haben wertvolle pharmakologische Eigenschaften. Durch Untersuchungen an der Spinalratte und am Kaninchen konnte festgestellt werden, dass die Verbindungen eine starke herzfrequenzsenkende Wirkung besitzen. Aufgrund dieser bradykarden Wirkung kommen sie für die Behandlung von Coronarerkrankungen in Frage. Es wurde z.B. gefunden, dass die Verbindung des nachstehend beschriebenen Beispiels 1 an der Spinalratte in einer Dosierung von 0,42 mg/kg die Herzfrequenz um 150 Schläge/min senkt.

Die Verbindungen der allgemeinen Formel I sowie deren Säureadditionssalze können enteral oder auch parenteral angewandt werden. Die Dosierung für die orale Anwendung liegt bei 0,1 bis 100 mg, vorzugsweise 0,5 bis 50 mg. Die Verbindungen der Formel I bzw. ihre physiologisch verträglichen Säureadditionssalze können auch mit andersartigen Wirkstoffen zum Einsatz gelangen. Geeignete galenische Darreichungsformen sind beispielsweise Tabletten, Kapseln, Zäpfchen, Lösungen, Emulsionen oder Pulver; hierbei können zu deren Herstellung die üblicherweise verwendeten galenischen Hilfs- oder Trägerstoffe, Spreng- oder Schmiermittel oder Substanzen zur Erzielung einer Depotwirkung Anwendung finden. Die Herstellung solcher galenischer Darreichungsformen erfolgt auf übliche Weise nach den bekannten Fertigungsmethoden.

Die folgenden Beispiele erläutern die Erfindung.

*Beispiel 1:*

*7-(2,6-Dibromphenyl)-2,3-dihydro-5-methyl-imidazo-[1,2-a]-imidazol (Verfahren a).*

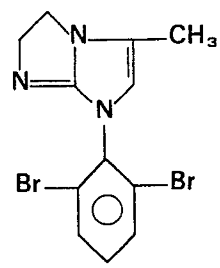

9,6 g (0,03 mol) 2-(2,6-Dibromphenylimino)-imidazolin werden zusammen mit 2,6 ml (110%) Chloraceton und 3 ml Triäthylamin in 30 ml absolutem Toluol im Rohr 17 h lang auf 150°C erhitzt. Hierauf dekantiert man die Toluolphase ab und löst den Rückstand in verdünnter Salzsäure. Bei aufsteigenden pH-Werten (Alkalisieren mit 2N Natronlauge) wird anschliessend fraktioniert mit Äther extrahiert. Die dünnschichtchromatographisch einheitlichen Ätherfraktionen werden vereinigt, über wasserfreiem Calciumsulfat getrocknet und der Äther im Vakuum abgezogen. Im Rückstand verbleiben 1,65 g entsprechend 15,4% der Theorie. Schmp.: 144 bis 147°C.

*Beispiel 2:*

*7-(2,6-Dichlorphenyl)-2,3-dihydro-5-methyl-imidazo-[1,2-a]-imidazol (Verfahren b).*

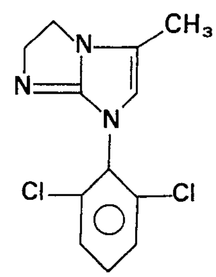

5,4 g 2-[N-(2,6-Dichlorphenyl)-N-(progargyl)amino]-2-imidazolin werden zusammen mit 5 Tropfen einer Trimethylbenzylammoniumhydroxydlösung in 40 ml absolutem Äthanol 5 h lang unter Rühren am Rückfluss erhitzt. Hierauf wird im Vakuum das Lösungsmittel abgezogen und der verleibende Rückstand in verdünnter Salzsäure gelöst. Bei aufsteigenden pH-Werten (Alkalisieren mit 2N Natronlauge) wird sodann fraktioniert mit Äther extrahiert. Die dünnschichtchromatographisch einheitlichen Ätherfraktionen um pH 9 werden vereinigt, über $MgSO_4$ getrock-

net und der Äther im Vakuum abgedampft. Im Rückstand verbleibt 1 g des neuen Imidazo-[1,2-a]-imidazols (18,7% der Theorie). Schmp.: 113 bis 117°C.

Entsprechend dem Beispiel 1 wurden die in Tabelle 2 aufgeführten Imidazol-[1,2-a]-imidazole der Formel VIII synthetisiert. Die Schmelzpunkte beziehen sich auf die Basen. Andernfalls ist die Salzform angegeben.

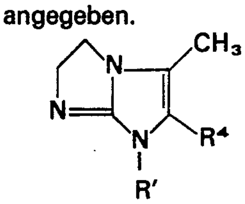

VIII

| Beispiel Nr. | R' | $R^4$ | Fp. (°C) | Ausbeute (% der Theorie) |
|---|---|---|---|---|
| 3 | 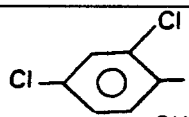 | H | 129-130 | 15,2 |
| 4 | 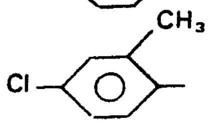 | H | Öl | 24,0 |
| 5 | 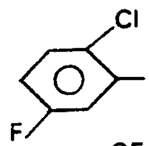 | H | 113-116 | 17,2 |
| 6 | 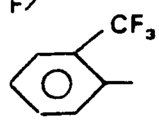 | H | Öl | 16,2 |
| 7 | 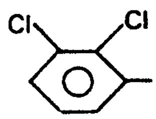 | H | 128-130 | 14,9 |
| 8 | 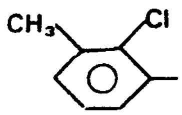 | H | 82-84 | 21,5 |
| 9 | 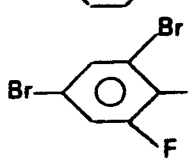 | H | 79-84 | 6,2 |
| 10 | 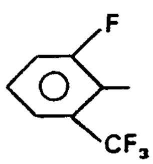 | H | 86-91 | 19,8 |
| 11 | 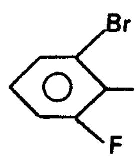 | H | 60-65 | 20,3 |
| 12 | 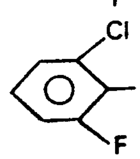 | H | 67-72 | 28,5 |
| 13 | 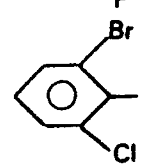 | H | 139-141 | 30,7 |

| Beispiel Nr. | R' | $R^4$ | Fp. (°C) | Ausbeute (% der Theorie) |
|---|---|---|---|---|
| 14 | 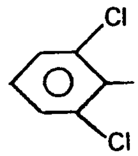 | $CH_3$ | 151-154 | 14,8 |
| 15 | 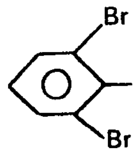 | $CH_3$ | 149-152 | 8,5 |
| 16 | 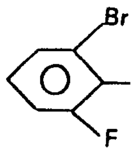 | $CH_3$ | 143-145 | 22,0 |
| 17 | 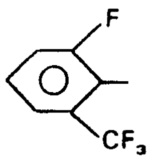 | $CH_3$ | 142-143 | 15,1 |
| 18 | 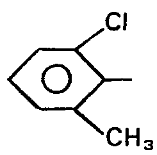 | $CH_3$ | Öl | 7,6 |
| 19 | 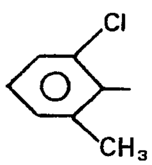 | H | Hydrobromid: 260 | 6,6 |

*Formulierungsbeispiele*

*Beispiel A: Tabletten*

| | |
|---|---|
| Wirkstoff gemäss Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:

Der Wirkstoff wird mit einem Teil der Hilfsstoffe vermischt, intensiv mit einer wässrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpresst, die dann in üblicher Weise mit Hilfe von Zucker, Talkum und Gummi arabicum dragiert werden.

*Beispiel B: Ampullen*

| | |
|---|---|
| Wirkstoff gemäss Erfindung | 1,0 mg |
| Natriumchlorid | 18,0 mg |
| dest. Wasser | ad 2,0 mg |

Herstellung:

Wirkstoff und Natriumchlorid werden in Wasser gelöst und unter Stickstoff in Glasampullen abgefüllt.

*Beispiel C: Tropfen*

| | |
|---|---|
| Wirkstoff gemäss Erfindung | 0,02 g |
| p-Hydroxybenzoesäuremethylester | 0,07 g |
| p-Hydroxybenzoesäurepropylester | 0,03 g |
| entmineralisiertes Wasser | ad 100 ml |

**Patentansprüche**

(für die benannten Vertragsstaaten: BE, CH, DE, FR, GB, IT, LU, NL, SE)

1. Imidazo-[1,2-a]-imidazole der allgemeinen Formel I:

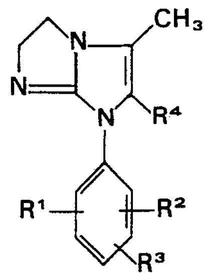

I

worin $R^1$, $R^2$ und $R^3$, die gleich oder verschieden sein können, ein Wasserstoffatom oder ein Fluor-, Chlor- oder Bromatom oder eine Methyl- oder Trifluormethylgruppe bedeuten, wobei mindestens einer der Reste $R^1$, $R^2$ oder $R^3$ nicht Wasserstoff sein darf, und $R^4$ ein Wasserstoffatom oder eine Methylgruppe bedeuten sowie deren physiologisch verträgliche Säureadditionssalze.

2. 7-(2,6-Dibromphenyl)-2,3-dihydro-5-methylimidazo-[1,2-a]-imidazol und dessen physiologisch verträgliche Säureadditionssalze.

3. Verfahren zur Herstellung von Verbindungen nach Anspruch 1, dadurch gekennzeichnet, dass man

a) ein substituiertes Phenyliminoimidazolin der allgemeinen Formel II:

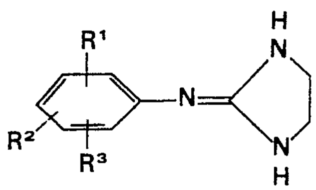

II

worin $R^1$ bis $R^3$ die oben genannten Bedeutungen besitzen, mit einer Oxoverbindung der allgemeinen Formel III:

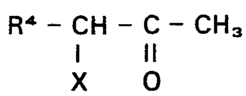

III

worin $R^4$ wie oben definiert ist und X ein Chlor-, Brom, oder Jodatom bedeutet, umsetzt, oder

b) ein 2-[N-Propargyl-N-phenylamino]-imidazolin-(2) der allgemeinen Formel IV:

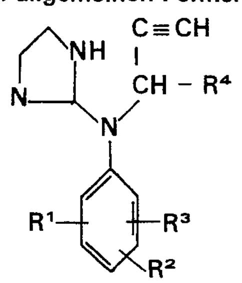

IV

worin $R^1$ bis $R^4$ die oben genannten Definitionen besitzen, cyclisiert, oder

c) aus einem 7-Phenyl-5-hydroxy-5-methyl-2,3,5,6-tetrahydroimidazo-[1,2-a]-imidazol der allgemeinen Formel V:

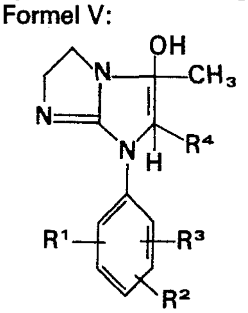

V

worin $R^1$ bis $R^4$ die oben angegebenen Bedeutungen haben, bei erhöhter Temperatur und/oder in Gegenwart wasserabspaltender Mittel Wasser abspaltet, oder

d) ein 1-Phenyl-2-imino-4-methylimidazolin-(4) der allgemeinen Formel VI:

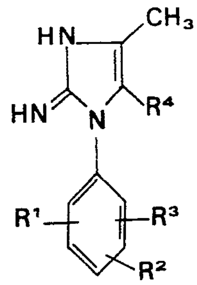

VI

worin $R^1$ bis $R^4$ wie oben angegeben definiert sind, mit 1,2-Dibromäthan umsetzt und die erhaltene Verbindung gewünschtenfalls in ein Säureadditionssalz überführt.

4. Pharmazeutische Zubereitungen, dadurch gekennzeichnet, dass sie eine oder mehrere Verbindungen nach Anspruch 1 als Wirkstoff enthalten.

5. Verfahren zur Herstellung von pharmaezeutischen Zubereitungen nach Anspruch 4, dadurch gekennzeichnet, dass man eine oder mehrere Verbindungen nach Anspruch 1 mit üblichen galenischen Hilfs- oder Trägerstoffen, Spreng- oder Schmiermitteln oder Substanzen zur Erzielung einer Depotwirkung verarbeitet.

6. Verbindungen der allgemeinen Formel I nach Anspruch 1 zur Anwendung bei der Behandlung von Coronarerkrankungen.

**Patentansprüche**

(für den benannten Vertragsstaat AT)

1. Verfahren zur Herstellung von Imidazo-[1,2-a]-imidazolen der allgemeinen Formel I:

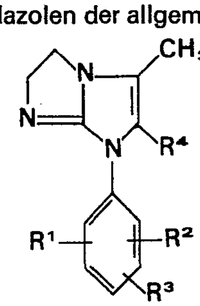

I

worin $R^1$, $R^2$ und $R^3$, die gleich oder verschieden sein können, ein Wasserstoffatom oder ein Fluor-, Chlor- oder Bromatom oder eine Methyl- oder Trifluormethylgruppe bedeuten, wobei mindestens einer der Reste $R^1$, $R^2$ oder $R^3$ nicht Wasserstoff sein darf, und $R^4$ ein Wasserstoffatom oder eine Methylgruppe bedeuten sowie von deren physiologisch verträglichen Säureadditionssalzen, dadurch gekennzeichnet, dass man

a) ein substituiertes Phenyliminoimidazolidin der allgemeinen Formel II:

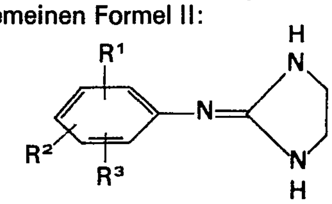

II

worin $R^1$ bis $R^3$ die oben genannten Bedeutungen besitzen, mit einer Oxoverbindung der allgemeinen Formel III:

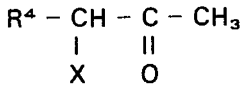 III

worin $R^4$ wie oben definiert ist und X ein Chlor-, Brom- oder Jodatom bedeutet, umsetzt, oder

b)ein 2-[N-Propargyl-N-phenylamino]-imidazolin-(2) der allgemeinen Formel IV:

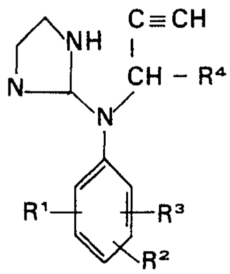 IV

worin $R^1$ bis $R^4$ die oben genannten Definitionen besitzen, cyclisiert, oder

c) aus einem 7-Phenyl-5-hydroxy-5-methyl-2,3,5,6-tetrahydroimidazo-[1,2-a]-imidazol der allgemeinen Formel V:

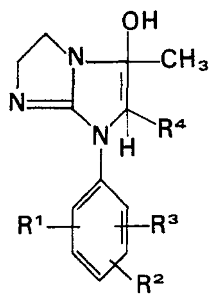 V

worin $R^1$ bis $R^4$ die oben angegebenen Bedeutungen haben, bei erhöhter Temperatur und/oder in Gegenwart wasserabspaltender Mittel Wasser abspaltet, oder

d) ein 1-Phenyl-2-imino-4-methylimidazolin-(4) der allgemeinen Formel VI:

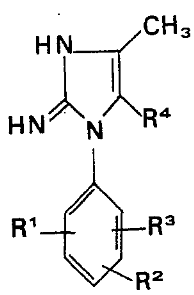 VI

worin $R^1$ bis $R^4$ wie oben definiert sind, mit 1,2-Dibromäthan umsetzt und die erhaltene Verbindung gewünschtenfalls in ein Säureadditionssalz überführt.

2. Verfahren zur Herstellung von 7-(2,6-Dibromphenyl)-2,3-dihydro-5-methylimidazo-[1,2-a]-imidazol und von dessen Säureadditionssalzen nach einem der Verfahren a bis d nach Anspruch 1.

3. Verfahren zur Herstellung von pharmazeutischen Zubereitungen, dadurch gekennzeichnet, dass man eine oder mehrere der nach Anspruch 1 hergestellten Verbindungen mit üblichen galenischen Hilfs- oder Trägerstoffen, Spreng- oder Schmiermitteln oder Substanzen zur Erzielung einer Depotwirkung verarbeitet.

## Claims

(for the contracting States BG, CH, DE, FR, GB, IT, LU, NL, SE)

1. Imidazo-[1,2-a]-imidazoles of general formula I:

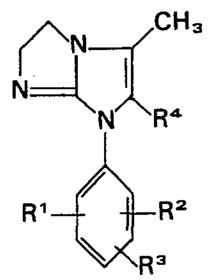 I

wherein $R^1$, $R^2$ and $R^3$, which may be the same or different, represent a hydrogen atom or a fluorine, chlorine or bromine atom or a methyl or trifluoromethyl group, whilst at least one of the groups $R^1$, $R^2$ or $R^3$ must not be hydrogen, and $R^4$ represents a hydrogen atom or a methyl group, and the physiologically acceptable acid addition salts thereof.

2. 7-(2,6-Dibromophenyl)-2,3-dihydro-5-methylimidazo-[1,2-a]-imidazole and the physiologically acceptable acid addition salts thereof.

3. Process for preparing compounds as claimed in claim 1, characterised in that

a) a substituted phenylimino-imidazolidine of general formula II:

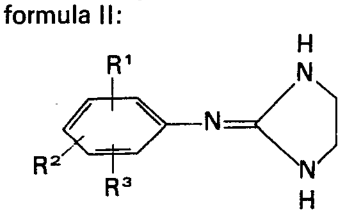 II

wherein $R^1$ to $R^3$ are as hereinbefore defined, is reacted with an oxo compound of general formula III:

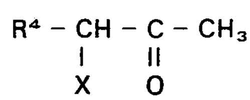 III

wherein $R^4$ is as hereinbefore defined and X represents a chlorine, bromine or iodine atom, or

b) a 2-[N-propargyl-N-phenylamino]-imidazolin-(2) of general formula IV:

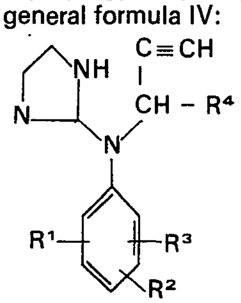 IV

wherein $R^1$ to $R^4$ are as hereinbefore defined, is cyclised, or

c) water is split off from a 7-phenyl-5-hydroxy-5-methyl-2,3,5,6-tetrahydro-imidazo-[1,2-a]-imidazole of general formula V:

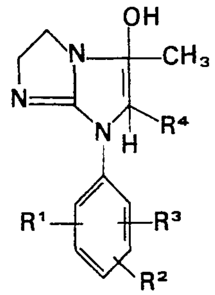

V

wherein $R^1$ to $R^4$ are as hereinbefore defined, at elevated temperature and/or in the presence of water-splitting off agents, or

d) a 1-phenyl-2-imino-4-methyl-imidazoline-(4) of general formula VI:

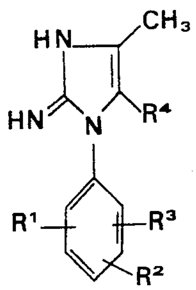

VI

wherein $R^1$ to $R^4$ are as hereinbefore defined, is reacted with 1,2-dibromoethane and, if desired, the compound obtained is converted into an acid addition salt.

4. Pharmaceutical preparations, characterised in that they contain one or more compounds as claimed in claim 1 as the active ingredient.

5. Process for preparing pharmaceutical preparations as claimed in claim 4, characterised in that one or more compounds as claimed in claim 1 are processed with conventional galenic excipients or carriers or disintegrants or lubricants or substances for obtaining delayed release.

6. Compounds of general formula I as claimed in claim 1 for use in the treatment of coronary diseases.

## Claims

(for the contracting State AT)

1. Process for preparing imidazo-[1,2-a]-imidazoles of general formula I:

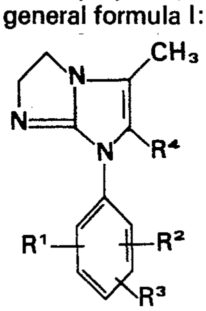

I

wherein $R^1$, $R^2$ and $R^3$, which may be the same or different, represent a hydrogen atom or a fluorine, chlorine or bromine atom or a methyl or trifluoromethyl group, whilst at least one of the groups $R^1$, $R^2$ or $R^3$ must not be hydrogen, and $R^4$ represents a hydrogen atom or a methyl group, and the physiologically acceptable acid addition salts thereof, characterised in that

a) a substituted phenylimino-imidazolidine of general formula II:

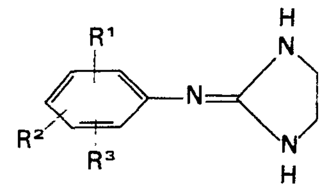

II

wherein $R^1$ to $R^3$ are as hereinbefore defined, is reacted with an oxo compound of general formula III:

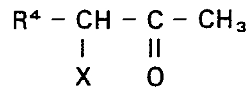

III

wherein $R^4$ is as hereinbefore defined and X represents a chlorine, bromine or iodine atom, or

b) a 2-[N-propargyl-N-phenylamino]-imidazoline-(2) of general formula IV:

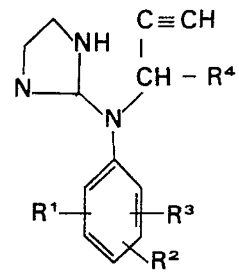

IV

wherein $R^1$ to $R^4$ are as hereinbefore defined, is cyclised, or

c) water is split off from a 7-phenyl-5-hydroxy-5-methyl-2,3,5,6-tetrahydro-imidazo-[1,2-a]-imidazole of general formula V:

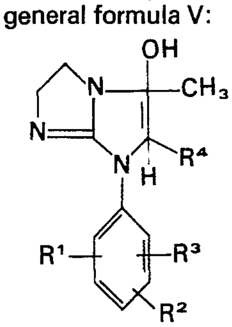

V

wherein $R^1$ to $R^4$ are as hereinbefore defined, at elevated temperature and/or in the presence of water-splitting off agents, or

d) a 1-phenyl-2-imino-4-methylimidazoline-(4) of general formula VI:

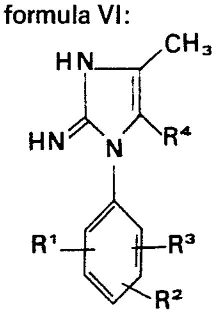

VI

wherein $R^1$ to $R^4$ are as hereinbefore defined, is reacted with 1,2-dibromoethane and, if desired, the compound obtained is converted into an acid addition salt.

2. Process for preparing 7-(2,6-dibromophenyl)-2,3-dihydro-5-methylimidazo-[1,2-a]-imidazole and the acid addition salts thereof, according to one of the processes a to d as claimed in claim 1.

3. Process for preparing pharmaceutical preparations, characterised in that one or more of the compounds prepared according to claim 1 are processed with conventional galenic excipients or carriers or disintegrants or lubricants or substances for obtaining delayed release.

**Revendications**

(Pour les Etats contractants BE, CH, DE, FR, GB, IT, LU, NL, SE).

1. Imidazo-[1,2-a]-imidazoles de formule générale I:

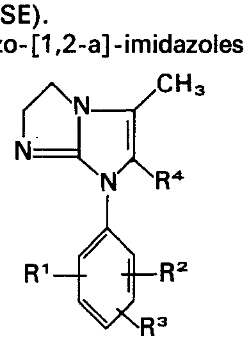

I

dans laquelle $R^1$, $R^2$ et $R^3$, qui peuvent être identiques ou différents, représentent un atome d'hydrogène ou un atome de fluor, de chlore ou de brome, ou un groupe méthyle ou trifluorométhyle, au moins l'un des radicaux $R^1$, $R^2$ ou $R^3$ ne pouvant pas être l'hydrogène, et $R^4$ signifie un atome d'hydrogène ou un groupe méthyle, ainsi que leurs sels physiologiquement supportables d'addition avec des acides.

2. Le 7-(2,6-dibromophényl)-2,3-dihydro-5-méthylimidazo-[1,2-a]-imidazole et ses sels physiologiquement supportables d'addition avec des acides.

3. Procédé pour la préparation des composés selon la revendication 1, caractérisà en ce que

a) on fait réagir une phénylimino-imidazolidine substituée de formule générale II:

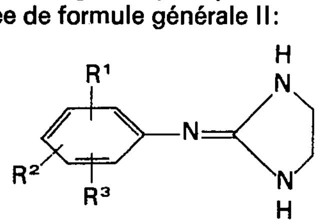

II

où $R^1$ à $R^3$ possèdent les significations mentionnées plus haut, avec un composé oxo de formule générale III:

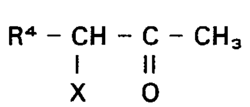

III

où $R^4$ est défini comme plus haut et X signifie un atome de chlore, de brome ou d'iode, ou

b) on cyclise une 2-[N-propargyl-N-phényl-amino]-imidazoline-(2) de formule générale IV:

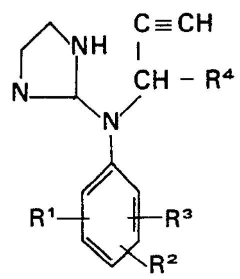

IV

où $R^1$ à $R^4$ possèdent les définitions mentionnées plus haut, ou

c) on élimine de l'eau à température augmentée et/ou en présence d'agent éliminant l'eau, à partir d'un 7-phényl-5-hydroxy-5-méthyl-2,3,5,6-tétrahydro-imidazo-[1,2-a]-imidazole de formule générale V:

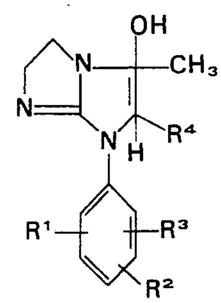

V

où $R^1$ à $R^4$ ont les significations données plus haut, ou

d) on fait réagir une 1-phényl-2-imino-4-méthylimidazoline-(4) de formule générale VI:

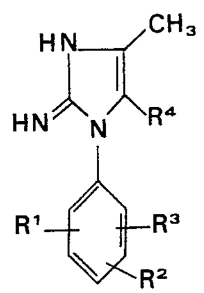

VI

où $R^1$ à $R^4$ sont définis comme indiqué plus haut, avec le 1,2-dibromoéthane, et on transforme éventuellement le composé obtenu en un sel d'addition avec un acide.

4. Préparations pharmaceutiques, caractérisées en ce qu'elles contiennent, en tant que substance active, un ou plusieurs composés selon la revendication 1.

5. Procédé pour la fabrication de préparations pharmaceutiques selon la revendication 4, caractérisé en ce qu'on traite un ou plusieurs composés selon la revendication 1 au moyen d'adjuvants ou excipients ou désagrégeants ou lubrifiants ou substances (pour obtenir un effet retard) galéniques habituels.

6. Composés de formule générale I selon la revendication 1 pour l'utilisation dans le traitement des maladies des coronaires.

**Revendications**

(Pour l'Etat contractant AT)

1. Procédé pour la préparation d'imidazo-[1,2-a]-imidazoles de formule générale I:

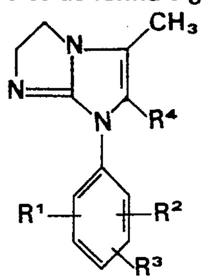

I

dans laquelle $R^1$, $R^2$ et $R^3$, qui peuvent être identiques ou différents, représentent un atome d'hydrogène ou un atome de fluor, de chlore ou de brome, ou un groupe méthyle ou trifluorométhyle, au moins l'un des radicaux $R^1$, $R^2$ ou $R^3$ ne pouvant pas être l'hydrogène, et $R^4$ signifie un atome d'hydrogène ou un groupe méthyle ainsi que leurs sels physiologiquement supportables d'addition avec des acides, caractérisé en ce que

a) on fait réagir une phénylimino-imidazolidine substituée de formule générale II:

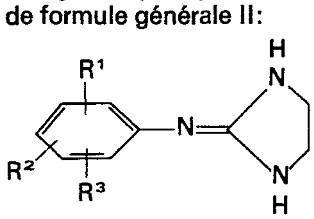

II

où $R^1$ à $R^3$ possèdent les significations indiquées plus haut, avec un composé oxo de formule générale III:

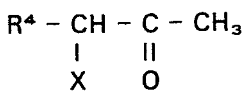

III

où $R^4$ est défini comme plus haut et X représente un atome de chlore, de brome ou d'iode, ou

b) on cyclise une 2-[N-propargyl-N-phényl-amino]-imidazoline-(2) de formule générale IV:

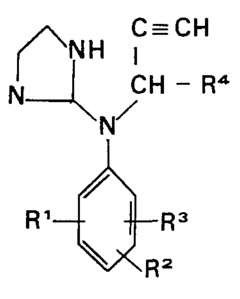

IV

où $R^1$ à $R^4$ possèdent les définitions indiquées plus haut, ou

c) on élimine de l'eau à température augmentée et/ou en présence d'un agent éliminant l'eau, à partir d'un 7-phényl-5-hydroxy-5-méthyl-2,3,5,6-tétrahydro-imidazo-[1,2-a]-imidazole de formule générale V:

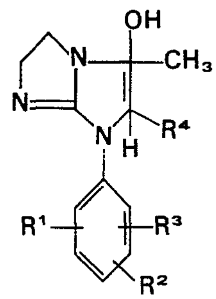

V

où $R^1$ à $R^4$ ont les significations indiquées plus haut, ou

d) on fait réagir une 1-phényl-2-imino-4-méthylimidazoline-(4) de formule générale VI:

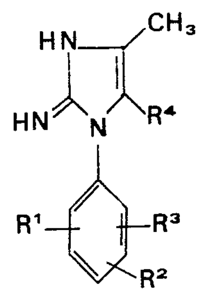

VI

où $R^1$ à $R^4$ sont définis comme plus haut, avec du 1,2-dibromoéthane, et on transforme éventuellement le composé obtenu en un sel d'addition avec un acide.

2. Procédé pour la préparation du 7-(2,6-dibromophényl)-2,3-dihydro-5-méthylimidazo-[1,2-a]-imidazole et de ses sels d'addition avec des acides selon l'un des procédés a à d selon la revendication 1.

3. Procédé pour la fabrication de préparations pharmaceutiques, caractérisé en ce qu'on traite un ou plusieurs composés préparés selon la revendication 1 au moyen d'adjuvants ou excipients ou désagrégeants ou lubrifiants ou substances (pour obtenir un effet retard) galéniques habituels.